# EUROPEAN PATENT SPECIFICATION

(11) **EP 2 392 254 B1**
(45) Date of publication and mention of the grant of the patent: **22.04.2020**
(21) Application number: 11168175.5
(22) Date of filing: 31.05.2011
(51) Int. Cl.: A61B 5/11

(54) **Patient position display system**
Patientenposition-Anzeigesystem
Système d'affichage de la position du patient

(30) Priority: 01.06.2010 JP 2010125984; 17.02.2011 JP 2011032271
(43) Date of publication of application: 07.12.2011
(73) Proprietor: Nihon Kohden Corporation, Shinjuku-ku Tokyo (JP)
(72) Inventor: Matsumura, Fumiyuki, Tokyo (JP); Tezuka, Shinji, Tokyo (JP); Muneshima, Yuichi, Tokyo (JP); Harasawa, Eishi, Tokyo (JP)
(74) Representative: Ilgart, Jean-Christophe

(56) References cited:
- WO-A1-03/082094
- WO-A1-2009/055635
- JP-A- 10 248 816
- JP-A- 2007 061 491
- US-A1- 2001 034 475
- US-A1- 2004 098 144

## Description

### BACKGROUND OF THE INVENTION

The present invention relates to a patient position display system which displays the position of the patient.

Various techniques for monitoring the state of the patient in a hospital have been known. In one of such techniques, the patient carries a medical transmitter, and the state of the patient is measured by the medical transmitter and transmitted to a remote display device. The state of the patient is displayed on the display device.

The patient can move in the hospital while carrying the medical transmitter. A technique has been known in which, in order to know the position of the patient, the position information is transmitted from the medical transmitter to the remote display device, and also the patient position is displayed on the display device (for example, see JP-A-10-248816).

In the technique disclosed in JP-A-10-248816, only when the medical transmitter is in communication with the display device, the patient position is displayed. Therefore, in the case where the patient carrying the medical transmitter moves outside the area in communication with the display device, for example, the patient position is immediately caused not to be displayed. Moreover, also in the case where the battery power of the medical transmitter is consumed and the communication with the display device is disabled, the patient position is not displayed. Since the patient position is not displayed in any way, there is no clue to the patient position.

Other patient patient position detection systems are disclosed in WO 2009/055635 A1, US 2001/034475 A1, US 2004/098144 A1, WO 03/082094 A1.

### SUMMARY

It is an object of the present disclosure to provide a patient position display system in which, even when a medical transmitter cannot communicate with a display device, it is possible to obtain a clue to the patient position.

In order to achieve the object, there is provided a patient position display system comprising: a position information transmitter which transmits position information on a position of a place; a medical transmitter which is to be attached to a patient and which detects and transmits biological information on the patient, the medical transmitter which can receive the position information from the position information transmitter and which, when receiving the position information, transmits the position information; an antenna which receives the position information and the biological information from the medical transmitter; and a display device which can display a position and a state of the patient based on the position information and biological information received by the antenna. Even when the antenna becomes not to receive the position information, the display device continues to display the position of the patient for a constant time period, based on position information which is last received.

According to the invention, there is provided a patient position display system according to independent claim 1. It comprises: a position information transmitter which transmits position information on a position of a place; a medical transmitter which is to be attached to a patient and which detects and transmits biological information on the patient, the medical transmitter which can receive the position information from the position information transmitter and which, when receiving the position information, transmits the position information; an antenna which receives the position information and the biological information from the medical transmitter; and a display device which displays a position and a state of the patient based on the position information and biological information received by the antenna. The display device selects to display or not to display the position of the patient. The display device determines whether the state of the patient is normal or abnormal, based on the biological information transmitted from the medical transmitter. When determining that the state of the patient is normal, the display device does not display the position of the patient, and when determining that the state of the patient is abnormal, the display device displays the position of the patient.

The position information transmitter may alternately repeat a transmission time period when the position information is transmitted, and a pause time period when the position information is not transmitted. The position information transmitter may be switchable between a gate mode in which the pause time period is shorter than a predetermined reference time period, and an area mode in which the pause time period is longer than the reference time period.

After the position information is received by the antenna, and before next position information is received, when the biological information becomes not to be received by the antenna within a predetermined time period, the display device may display that the medical transmitter moves outside a reception area of the antenna, and, when the biological information becomes not to be received by the antenna after an elapse of the predetermined time period, the display device may display that a power supply of the medical transmitter is lost.

According to the present disclosure, there is also provided a patient position display system comprising: a position information transmitter which transmits position information on a position of a place; a medical transmitter which is to be attached to a patient and which detects and transmits biological information on the patient, the medical transmitter which can receive the position information from the position information transmitter and which, when receiving the position information, transmits the position information; an antenna which receives the position information and the biological information from the medical transmitter; and a display device which can display a position and a state of the patient based on the position information and biological information received by the antenna. The display device stores the position information and the biological information in time sequence, and the display device displays the position and the state of the patient in time sequence based on the stored position information and biological information.

When the antenna continues to receive position information on a specific position for a predetermined time period, the display device may notify an audible alarm or displaying an image.

The medical transmitter may include a direction detection sensor which detects a traveling direction of the patient, and transmit information on the detected traveling direction of the patient together with the biological information. The display device may display the traveling direction of the patient based on the information on the traveling direction.

### BRIEF DESCRIPTION OF THE DRAWINGS

Fig. 1 is a diagram schematically showing the configuration of a patient position display system.
Fig. 2 is a view showing transmission timings in area and gate modes of a position information transmitter.
Fig. 3 is a block diagram schematically showing the configuration of a display device.
Fig. 4 is a diagram showing the manner in which the patient moves.
Fig. 5 is a view showing a display example in a first display mode.
Fig. 6 is a chart showing the operation of the display device.
Fig. 7 is a chart showing the operation of the display device which is performed, concurrently with the operation shown in Fig. 6.
Fig. 8 is a diagram showing the patient.
Fig. 9 is a view showing a display example in a second display mode.
Fig. 10 is a diagram showing the patient.
Fig. 11 is a view showing another display example in the second display mode.
Fig. 12 is a view showing display examples of an alarm.
Fig. 13 is a view showing a display example in a third display mode.
Fig. 14 is a diagram showing the manner of attaching a medical transmitter in a fourth display mode, and the action of the geomagnetism.
Fig. 15 is a view showing detection results of magnetic strengths in three axes by a geomagnetic sensor.
Fig. 16 is a view showing a display example in the fourth display mode.
Fig. 17 is a view showing another display example in the fourth display mode.
Fig. 18 is a view showing a display example in a fifth display mode.

### DETAILED DESCRIPTION OF EMBODIMENTS

Hereinafter, embodiments of the invention will be described with reference to the accompanying drawings. In the figures, the identical components are denoted by the same reference numerals and duplicated description will be omitted. In the drawings, the dimension ratios are exaggerated for the sake of convenience in description, and may be sometimes different from the actual ratios.

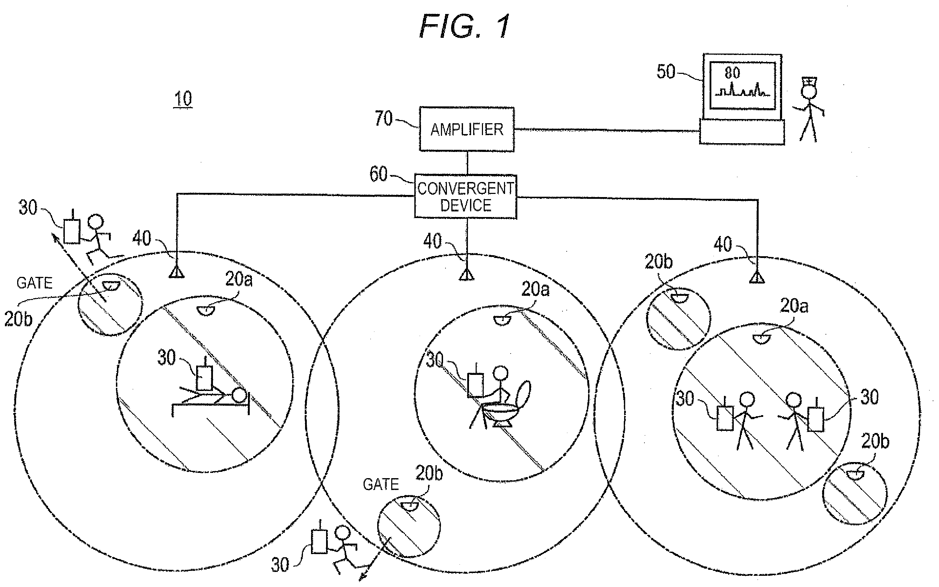

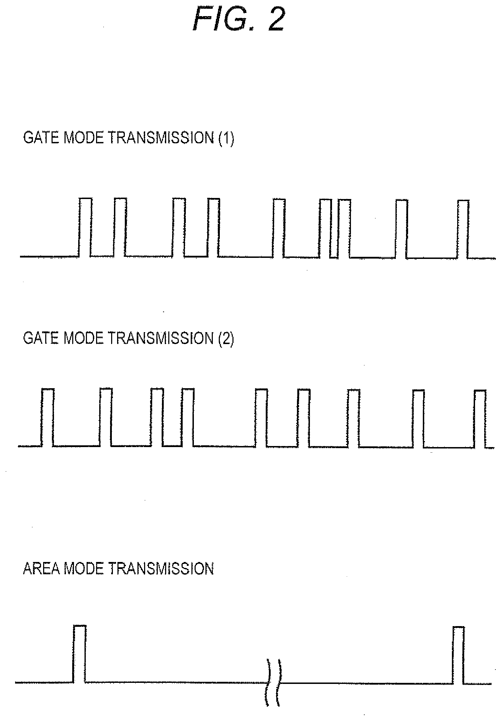

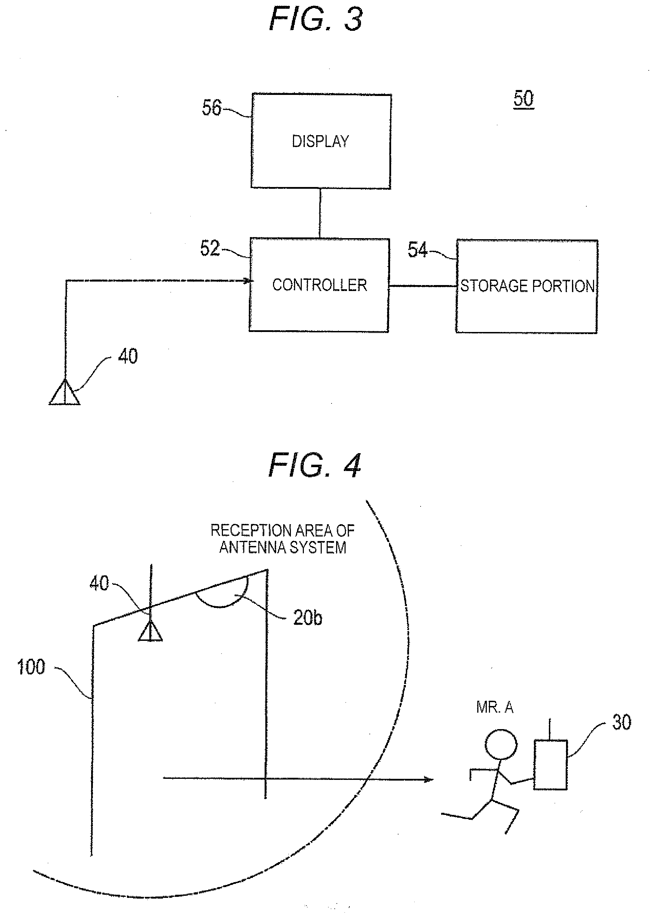

Fig. 1 is a diagram schematically showing the configuration of a patient position display system, Fig. 2 is a view showing transmission timings in area and gate modes of a position information transmitter, and Fig. 3 is a block diagram schematically showing the configuration of a display device.

The patient position display system 10 is used in, for example, a hospital. Hereinafter, a case where the patient position display system 10 is used in a hospital will be described.

As shown in Fig. 1, the patient position display system 10 has position information transmitters 20 (20a, 20b), medical transmitters 30, antennas 40, and a display device 50. Their configurations will be described.

### (Position information transmitter 20)

The position information transmitters 20 are disposed in various places of the hospital, such as medical wards, toilets, dayrooms, a dining hall, elevators, emergency exits, gates of such facilities, and corridors, respectively. An ID which is unique to each of the places is set in the position information transmitter 20 disposed in the place, and the ID is transmitted as position information.

Each of the position information transmitters 20 can be set (switched) to an area mode in which the transmitter covers a relatively wide range such as a medical ward, a toilet, or a dayroom, or to a gate mode in which the transmitter covers a relatively narrow range such as a gate or a corridor. In the figure, position information transmitters which are set to the area mode are denoted by 20a, and those which are set to the gate mode are denoted by 20b.

The position information transmitter 20a which is set to the area mode transmits position information at relatively long intervals (pause time period). Therefore, the position information transmitter 20a is disposed in a place where the patient stays for a long time period. In the area mode, for example, the position information transmitter 20a transmits position information at random intervals in a range of 10 to 13 sec.

The position information transmitter 20b which is set to the gate mode transmits position information at relatively short intervals (pause time period). Therefore, the position information transmitter 20b is disposed in a place through which the patient passes at once. For example, the position information transmitter 20b transmits position information at random intervals in a range of 0.1 to 0.5 sec. In a corridor or a gate, particularly, the patient may immediately pass through, and therefore two or more position information transmitters 20b are disposed together. According to the configuration, the position information transmitters 20b can complement each other in transmission timing of position information. The transmission intervals are randomly set. Even when the position information transmitters 20b start transmission at the same time, therefore, the transmission timings do not overlap each other as shown in gate mode transmissions (1) and (2) in Fig. 2.

As described above, in the gate mode and the area mode, their pause time periods when the signal is not transmitted are different from each other. The pause time periods are not restricted to the above-described examples. In the case of the gate mode, the pause time period can be randomly set within a range of a reference time period or shorter, and, in the area mode, similarly, it can be randomly set within a range of the reference time period or longer. The reference time period can be arbitrarily set in accordance with the environment where the transmitter is disposed. For example, the reference time period is 5 sec.

### (Medical transmitter 30)

Each of the medical transmitters 30 is an apparatus which can be carried by the patient, and is attached to the patient to detect and transmit biological signals of the patient. More specifically, the medical transmitter 30 has various biological information sensors such as a transducer for the arterial oxygen saturation, and a plurality of electrocardiogram electrodes (not shown), and detects electrocardiogram signals, heart rate, body temperature, arterial oxygen saturation, and respiratory rate of the patient as biological signals.

The medical transmitter 30 can further receive position information which is transmitted from the position information transmitter 20. Upon receiving the position information, the medical transmitter 30 transmits position information in addition to the biological signals.

In the figures, for the sake of clarity, the medical transmitter 30 is shown in a manner that the patient holds it in hand. Alternatively, in order to allow the patient to easily carry the medical transmitter 30, the transmitter may be attached to means which is to be secured to the body, such as a belt. In such a belt, for example, a case for holding the medical transmitter 30 is attached to the belt, and the medical transmitter 30 is fixed into the case. According to the configuration, the hands are prevented from being full, and the patient can comfortably act.

### (Antenna 40)

The antenna 40 receives the biological information and position information transmitted from the medical transmitter 30. As conceptually shown in Fig. 1, the antenna 40 covers an area including a range (hatched in the figure) where the position information transmitter 20 can transmit position information. The medical transmitter 30 receives the position information in the transmission range of the position information transmitter 20, and transmits it together with the biological information. Since the area covered by the antenna 40 is wider than the transmission range of the position information transmitter 20, the antenna 40 can receive the position information transmitted from the medical transmitter 30.

The antennas 40 which are disposed in a plurality of places are assembled by a convergent device 60. Sets of information which are received by the antennas 40 are assembled into the convergent device 60. The convergent device 60 is connected to an amplifier 70. The amplifier 70 amplifies the signals which are received by the antennas 40, and transmits the amplified signals to the display device 50.

### (Display device 50)

The display device 50, which is, for example, a central monitor, can display the position and state of the patient, based on the position information and biological information received by the antenna 40. The display device 50 has a controller 52, a storage portion 54, and a display 56.

As described above, the display device 50 receives the position information and the biological information through the convergent device 60 and the amplifier 70. The position information and the biological information are received by the controller 52. The controller 52 stores the received position and biological information in time, sequence in the storage portion 54. Furthermore, the controller 52 performs calculations so as to visualize the position and state of the patient, and then controls the display 56 so as to display the position and the state thereon.

Various methods may be employed as the method of displaying the position and state of the patient on the display 56, as described later. Hereinafter, such methods will be described as first to fifth display modes.

### (First display mode)

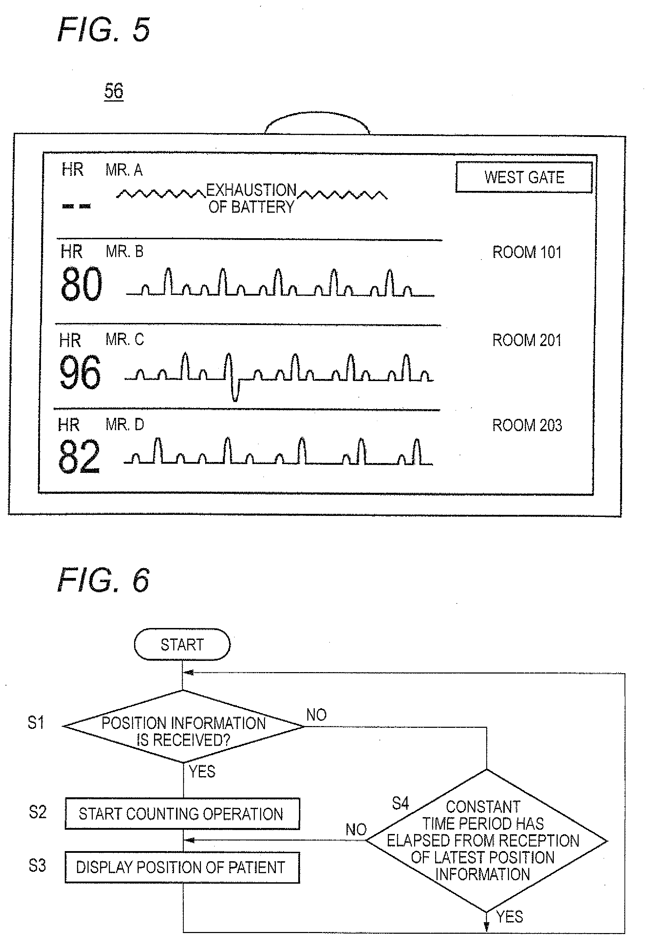

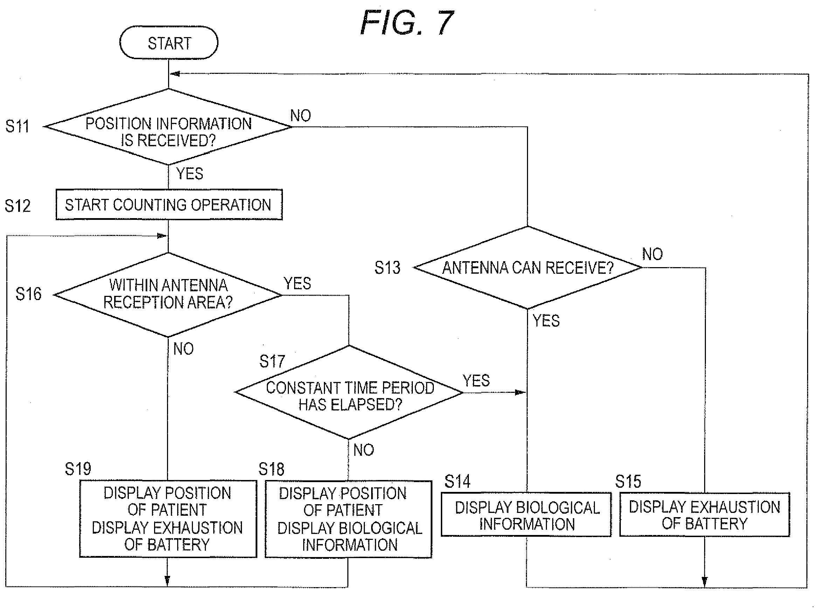

Fig. 4 is a diagram showing the manner in which the patient moves, Fig. 5 is a view showing a display example in the first display mode, Fig. 6 is a chart showing the operation of the display device, and Fig. 7 is a chart showing the operation of the display device which is performed concurrently with the operation shown in Fig. 6.

It is assumed that, as shown in Fig. 4, the patient (Mr. A) passes through a gate 100. One of the position information transmitters 20b which transmits an ID indicating the position of the gate 100 is disposed in the gate 100. Also one of of the antennas 4 is disposed in the gate 100. At this time, the display device 50 operates as shown in Fig. 6. Basically, the operation of the display device 50 is performed by the controller 52.

First, the display device 50 determines whether the position information is received from the medical transmitter 30 carried by the patient or not (step S1). When the patient just passes through the gate 100, the medical transmitter 30 carried by the patient enters the transmission range of the position information transmitter 20b, and receives the position information. The medical transmitter 30 transmits the received position information together with the biological information on the patient to the antenna 40.

If the display device 50 receives the position information through the antenna 40 (step S1: YES), the display device starts the operation of counting the time period from the reception of the latest position information (step S2) . Based on the received position information, the display device 50 displays the position of the patient on the display 56 (step S3). As shown in Fig. 5, for example, the positions of the respective patients are displayed in the right portion of the screen. The control of the display device 50 returns to the process of step S1 to again check whether the position information is received or not.

If the patient passes over the transmission range of the position information transmitter 20b and the display device 50 cannot receive the position information (step S1: NO), the display device 50 determines whether a constant time period has elapsed from the reception of the latest position information or not (step S4). The constant time period is a time period when, even in the case where the medical transmitter 30 of the patient cannot receive the position information, the last position of the patient is displayed. The constant time period can be arbitrarily set, and maybe set to, for example, 30 sec.

If the constant time period has not elapsed (step S4: NO), the control proceeds to step S3 where the display device 50 continues to display the position of the patient based on the last received position information, on the display 56. In order to distinguish the position from that which is displayed as result of reception of the current position information, the position display may be enclosed by a rectangle as in "West gate" in Fig. 5, or "west gate" may be blinkingly displayed.

By contrast, if the constant time period has elapsed (step S4: YES), the latest position of the patient is not known, and therefore the position is not displayed. Then, the control returns to the process of step S1.

In the first display mode, as described above, the position of the patient is continued to be displayed for the constant period time. Even when an uncommunicable state occurs because a battery power for the medical transmitter is consumed or the patient moves outside the communication range of the position information transmitter, therefore, it is possible to know the latest position of the patient by using the position of the patient as a clue.

In the operation shown in Fig. 6, even when the position information cannot be received, the position information is held for the constant time period to be displayed as the latest patient position. However, the operation is not limited to this. Alternatively, in the case where the position information cannot be received, the latest patient position may be continued to be displayed, based on the position information on the patient which is last received, until the next position information is received.

The display device 50 performs also the operation shown in Fig. 7, concurrently with that shown in Fig. 6.

The display device 50 determines whether the position information is received through the antenna 40 or not (step S11). If the display device 50 receives the position information (step S11: YES), the display device starts the operation of counting the time period from the reception of the position information (step S12). The constant time period is a time period which is arbitrarily set.

If the display device 50 dose not receive the position information (step S11: NO), the display device 50 determines whether, even though the patient is within the antenna reception area and the position information is not received, the biological information is received or not (step S13). When the patient is not in the transmission range of the position information transmitter 20, the display device 50 does not receive the position information, but, when the patient is in the reception area of the antenna 40, can receive the biological information.

If the patient is within the antenna reception area and the biological information is received (step S13: YES), it is confirmed that the patient is at least in the reception area of the antenna 40 and the medical transmitter 30 normally operates. "Biological information" is displayed (step S14), and then the control returns to the process of step S11. The process from step S11 to step S14 indicates that the patient is in the antenna reception area.

If the reception is not performed through the antenna (step S13: NO), the display device 50 displays information indicating that the power supply of the medical transmitter 30 is lost, such as "Interruption of radio wave" or "Interruption of radio wave due to exhaustion of battery" on the display 56 (step S15). The process from step S11 to step S15 indicates that, for example, although the patient is in the antenna reception area, the transmitter stops the transmission of the radio wave because of exhaustion of the battery or a failure in the transmitter, and reception cannot be performed.

After the counting operation starts (step S12), the display device 50 determines whether the patient is within the antenna reception area and biological information is received or not (step S16). If biological information is received (step S16: YES), it is known that the patient is within the antenna reception area, and the display device 50 determines whether a constant time period has elapsed from the start of the counting operation (step S17). If the constant time period has not elapsed (step S17: NO), the display device 50 displays "Position of patient" and "Biological information" (step S18). Then, the control returns to the process of step S16. The process from step S11 to step S17 indicates that the patient has passed through the gate but is within the antenna reception area, for example, the case where the patient has passed through the gate from a place other than the reception area and returned into the antenna reception area.

If the constant time period has elapsed (step S17: YES), the display device 50 displays "Biological information" (step S14). Then, the control returns to the process of step S11. The process from step S11 to step S14 via step S17 indicates that the patient has passed through the gate but is within the antenna reception area, for example, the case where the patient has passed through the gate from a place other than the reception area and returned into the antenna reception area, and the constant time period has elapsed from the returning.

If, after the counting operation starts (step S12), biological information cannot be received (step S16: NO), it is known that the patient is outside the antenna reception area, and the display device 50 displays "Exhaustion of battery" and holds the display of "Position of patient" (step S19). Then, the control returns to the process of step S16. The process from step S11 to step S19 indicates that, for example, the patient has passed through the gate and moved outside the antenna reception area.

### (Second display mode)

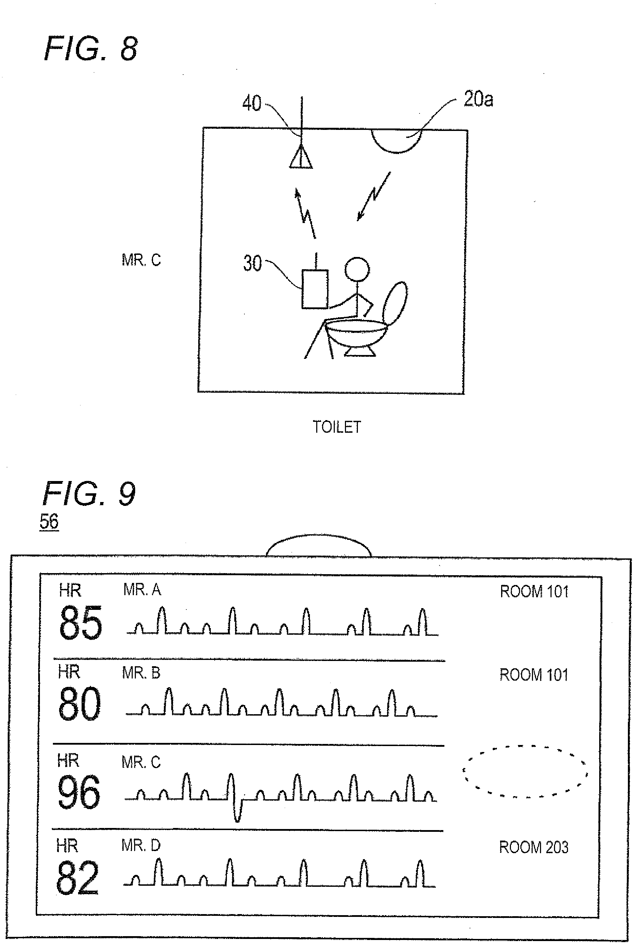

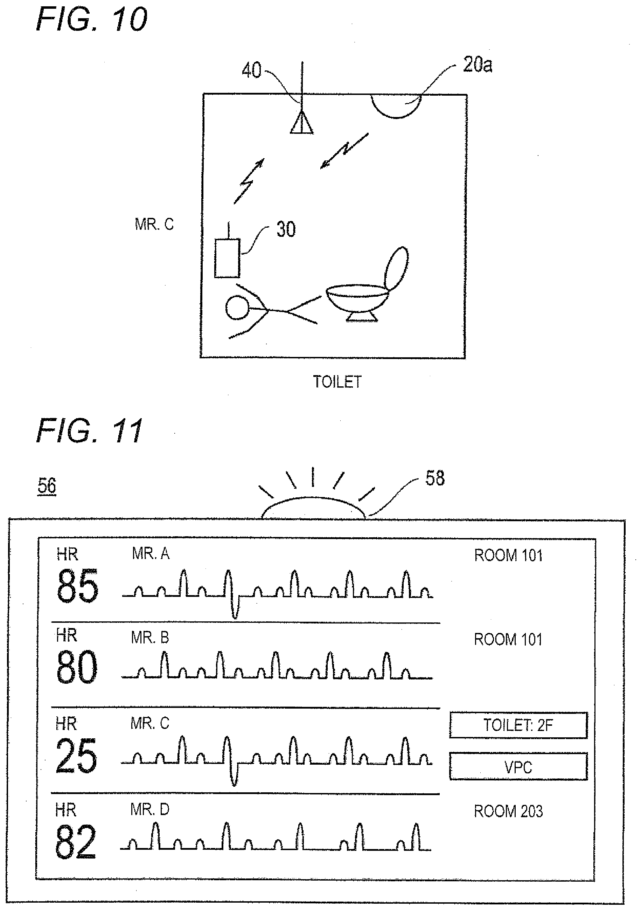

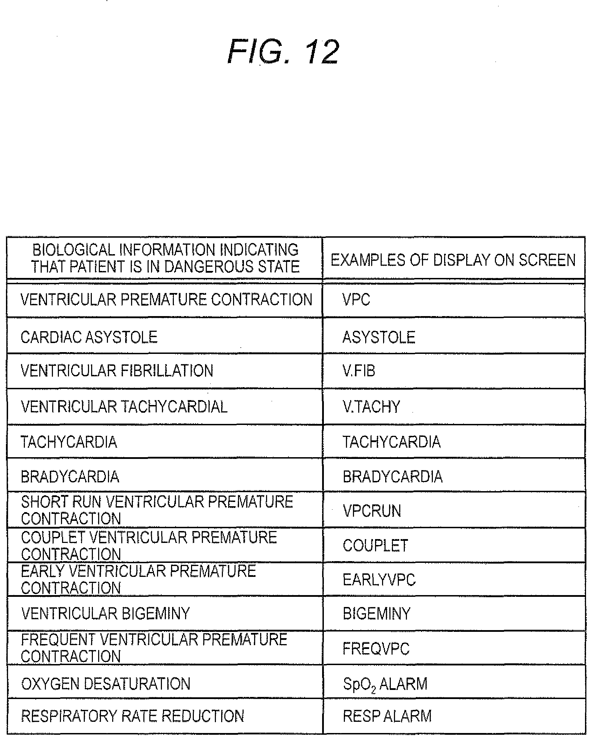

Fig. 8 is a diagram showing the patient, Fig. 9 is a view showing a display example in the second display mode, Fig. 10 is a diagram showing the patient, Fig. 11 is a view showing another display example in the second display mode, and Fig. 12 is a view showing display examples of an alarm.

In the second display mode, the display device 50 can select the display or non-display of the position of the patient. In the case where the patient is in an abnormal state, however, the position of the patient is displayed irrespective of the selection of the display or the non-display.

In the display device 50, with respect to an arbitrary place, the display or the non-display can be set through an inputting device such as a keyboard, a mouse, or a touch panel. When the patient is in a toilet, for example, an operator can select the non-display.

Here, it is assumed that Mr. C is in the toilet as shown in Fig. 8. As indicated by the broken line oval in Fig. 9, then, the location of Mr. C is not displayed. As described above, with respect to an arbitrary place, it is possible to daringly select the non-display of the position on the display device 50 even in the case where the position information on the patient is received. According to the configuration, the privacy of the patient can be ensured.

In the case where an abnormality occurs in Mr. C as shown in Fig. 10, however, the location (the toilet: 2F) is displayed on the display 56 of the display device 50 as shown in Fig. 11. The abnormality of the state of Mr. C is found by detection of biological information by the medical transmitter 30. As described above, the medical transmitter 30 always monitors the biological information on the patient, and transmits the information to the display device 50 through the antenna 40. Therefore, the controller 52 of the display device 50 can determine an abnormality of the patient based on the received biological information. If the display device 50 determines that an abnormality occurs, a warning light 58 of the display 56 may be turned on so that the abnormality is further visually clarified.

As shown in Fig. 11, the display device 50 may further display the symptom of the patient, based on the received biological information. In the case of a ventricular premature contraction, for example, "VPC" is displayed on the display device 50.

Examples of other symptoms are listed in Fig. 12. In the case of a symptom indicated in the left column of Fig. 12, the signs shown in the right column are displayed on the display device 50.

In the case of the second display mode, when it is determined that an abnormality occurs, as described above, the position of the patient is displayed. While ensuring the privacy of the patient when the state of the patient is normal, therefore, it is possible to know the position of the patient when an abnormality occurs, and emergency response can provided. Moreover, also the symptom is displayed, and hence preparation for rush to the location of the patient can be performed in advance.

The second display mode may be combined with the first display mode, or may be singly applied to the patient position display system 10.

### (Third display mode)

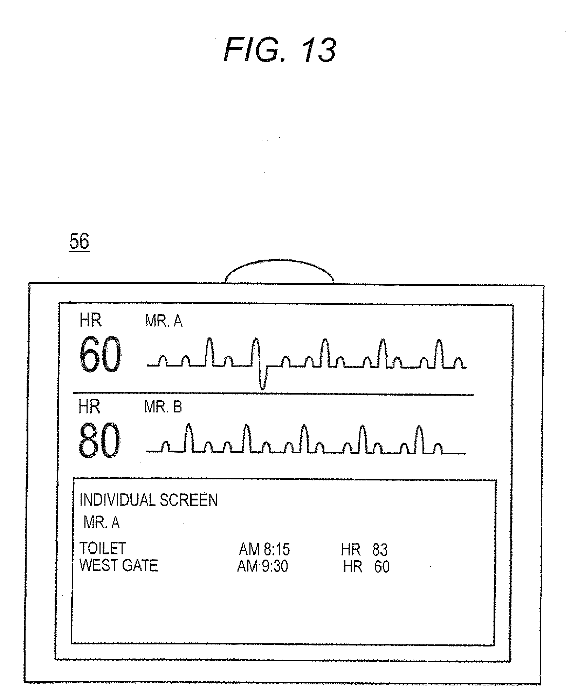

Fig. 13 is a view showing a display example in the third display mode.

In the third display mode, the display device 50 stores the received position and biological information in time sequence in the storage portion 54, and displays the position and state of the patient in time sequence based on the stored position and biological information.

As described above, position information and biological information on the patient are monitored, and transmitted to the display device 50 through the antenna 40. The display device 50 displays the state and location of the patient in real time on the display 56, and can store the state and location of the patient in time sequence while being correlated with the time. The stored information in time sequence is displayed as required on the display 56. In the case where the location and health condition of the patient Mr. A from the morning are to be seen, for example, the display device 50 displays information on Mr. A on the display 56 as shown in Fig. 13. Since the position and state of the patient can be displayed in time sequence, the state of the patient can be analyzed in more detail.

The third display mode may be combined with the first display mode or the second display mode, or may be singly applied to the patient position display system 10.

### (Fourth display mode)

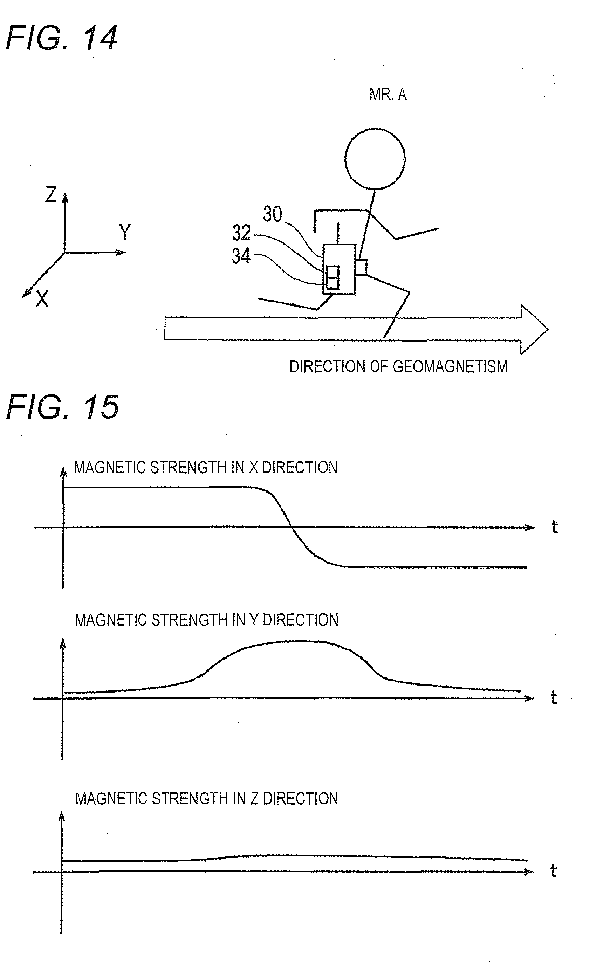

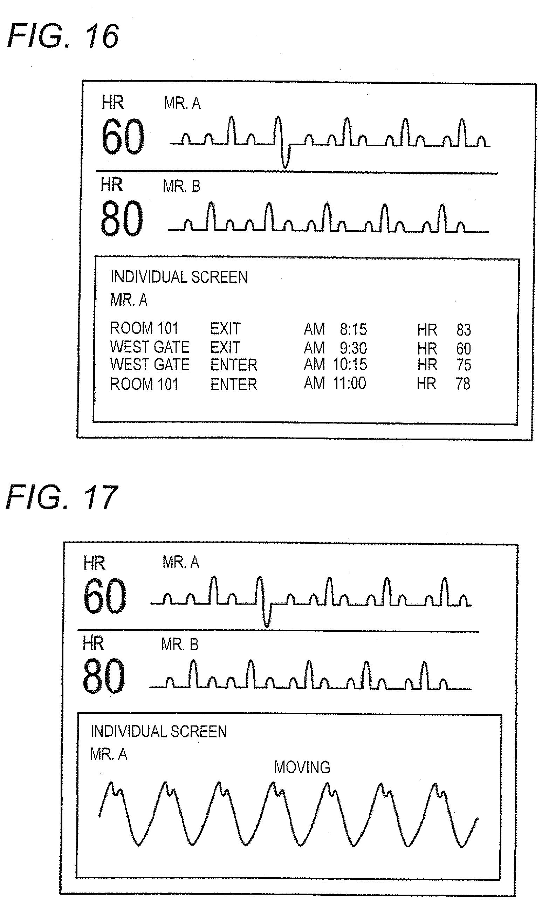

Fig. 14 is a diagram showing the manner of attaching a medical transmitter in the fourth display mode, and the action of the geomagnetism, Fig. 15 is a view showing detection, results of magnetic strengths in three axes by a geomagnetic sensor, Fig. 16 is a view showing a display example in the fourth display mode, and Fig. 17 is a view showing another display example in the fourth display mode.

In the fourth display mode, the geomagnetic sensor 32 and an acceleration sensor 34 are added to the medical transmitter 30, and the direction and movement of the patient are detected and displayed on the display device 50.

The medical transmitter 30 for the fourth display mode has the geomagnetic sensor 32 as a direction detection sensor. The geomagnetic sensor 32 detects the geomagnetism to measure the orientation. In the embodiment, the sensor detects three axes of the lateral direction (X direction), the longitudinal direction (Y direction), and the vertical direction (Z direction). It is assumed that, in Fig. 14, the Y direction is in parallel to the geomagnetism, the X direction is orthogonal to the geomagnetism in a horizontal plane, and the Z direction is orthogonal to the geomagnetism in a vertical plane. The geomagnetic sensor 32 detects magnetic strengths in the X, Y, and Z directions, whereby the direction of the medical transmitter 30 to which the sensor is attached, i.e., that of the patient can be calculated.

As shown in Fig. 15, at the timing when, viewed in time sequence, the magnetic strength in the X direction is 0 and that in the Y direction is at the maximum, for example, the patient is oriented in the direction of the geomagnetism. The Z direction indicates the height. When the patient walks on a flat floor, therefore, the magnetic strength in the Z direction does not fluctuate. The calculation of the direction of the geomagnetic sensor 32 based on the magnetic strengths in the axes is well known, and therefore a detailed description thereof is omitted.

In the case where the direction (traveling direction) of the patient is to be measured by the geomagnetic sensor 32, the attitude of the geomagnetic sensor 32 must be fixed. Therefore, the medical transmitter 30 is secured to the body of the patient by the above-described means such as a belt so that the attitude of the geomagnetic sensor 32 is constant. For example, the medical transmitter 3 0 is held into the case fixed to the belt.

The direction of the patient which is measured by the geomagnetic sensor 32 is transmitted as traveling direction information on the patient from the medical transmitter 30 to the antenna 40. The traveling direction information on the patient which is transmitted to the antenna 40 is further transmitted together with biological information to the display device 50. At the timing when the patient passes through the gate 100 in Fig. 4, for example, also the traveling direction information on the patient is transmitted to the display device 50 through the antenna 40. When the direction of the patient at passing through the gate 100 is found, it is possible to know whether the patient is to exit the room or to enter the room.

The display device 50 can display the entrance/exit of the patient on the display 56 as shown in, for example, Fig. 16. In Fig. 16, it is displayed that the patient Mr. A exits Room 101 at 8:15, exits the west gate at 9:30, returns to the west gate at 10:15, and returns to Room 101 at 11:00. In the fourth display mode, as described above, not only the passage of the patient through the gate 100 or the like, but also the direction of the passage can be known. Therefore, the position of the patient can be traced more correctly. Even when an abnormality occurs in the patient, the position of the patient can be immediately identified.

Furthermore, the medical transmitter 30 has the acceleration sensor 34. The acceleration sensor 34 measures the acceleration. In the fourth display mode, also acceleration information (the output voltage) on the acceleration sensor 34 is transmitted together with the biological information to the display device 50 through the antenna 40. As shown in Fig. 17, for example, the acceleration information is displayed in time sequence. In the case where the output voltage of the acceleration sensor 34 is periodically varied as shown in Fig. 17, it is known that the patient is moving. The display device 50 may analyze the period of the output voltage to determine whether the patient is moving or not, and display a result on the display 56.

From the display on the display device 50, as described above, it is possible to know whether the patient is moving or stopping. When, even in the case where an abnormality is observed in the biological information, it is known that the patient continues to constantly move, therefore, it is possible to determine that the patient is normal and an abnormality occurs in a device such as the medical transmitter 30 or their attachment states.

The fourth display mode may be combined with the above-described first to third display modes, or may be singly applied to the patient position display system 10.

In the fourth display mode, the case where the geomagnetic sensor 32 can detect the three axes has been described. As far as at least the direction of the patient other than the height direction is known, however, the entrance/exit of the patient through a gate can be determined. Therefore, a two-axis geomagnetic sensor which can detect two axes of the X and Y directions may be used.

### (Fifth display mode)

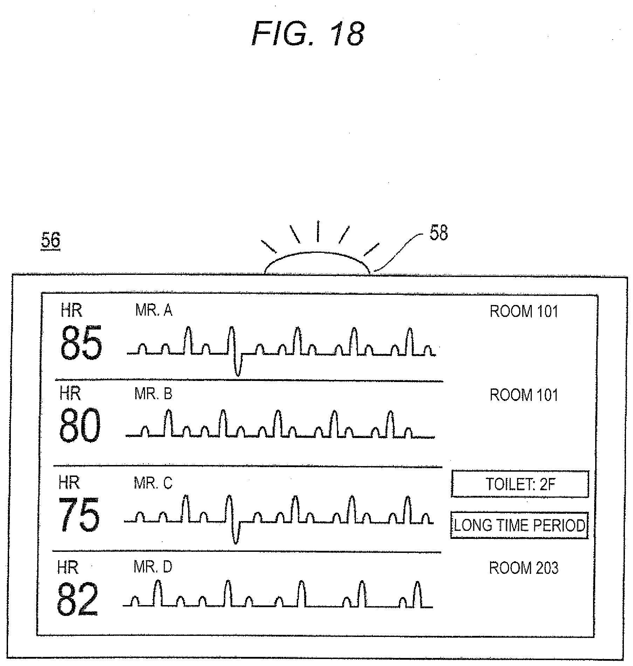

Fig. 18 is a view showing a display example in the fifth display mode.

In the fifth display mode, the display device 50 determines whether the patient is at a specific position for a predetermined time period or longer or not, based on the position information which is received through the antenna 40. The specific position is a place where the patient may possibly stay for a short time period, but does not stay for a long time period, such as a toilet or a dressing room. The predetermined time period is a time period when, although appropriately changed depending on the specific position, the patient does not stay at the specific position in a usual case. In the case where the specific position is a toilet, for example, the predetermined time period is set to 20 min., and in the case of a dressing room, set to 10 min.

In the case where the display device 50 receives position information related to the specific position through the antenna 40, the device measures the time period when the reception of the information related to the specific position is continued. When the position information on a toilet begins to be received, for example, the display device 50 measures the staying time period because the patient is in the toilet which is set as the specific position. When the measured time period exceeds the predetermined time period, e.g., 20 min., the display device 50 issues an alarm through the display 56 as shown in Fig . 18. The alarm may be performed by turning on the warning light 58, by visual notification such as a display of characters of "Long time period" or an image on a part of the display 56 where the position of the patient is indicated, or by a sound. In the case of a sound, for example, a message "Stay at a specific position for a long time period" is announced by the display device 50.

In the fifth display mode, when the patient stays at the specific position for the predetermined time period or longer, as described above, an alarm is issued. Even when an abnormality occurs in the patient and the patient cannot move, therefore, it is possible to find the abnormality in an early stage.

The fifth display mode may be combined with the above-described first to fourth display modes, or may be singly applied to the patient position display system 10.

According to an aspect of the disclosure, the patient position is continued to be displayed for a predetermined time period. Even when an uncommunicable state occurs because a battery power for the medical transmitter is consumed or the patient moves outside the communication range of the position information transmitter, therefore, it is possible to know the latest position of the patient.

## Claims

1. A patient position display system (10) comprising:
a position information transmitter (20a, 20b) adapted to transmit position information on a position of a place;
a medical transmitter (30) attachable to a patient and adapted to detect and transmit biological information on the patient, the medical transmitter being adapted to receive the position information from the position information transmitter and to transmit the position information when receiving the position information;
an antenna (40) adapted to receive the position information and the biological information from the medical transmitter; and
a display device (50) adapted to display a position and a state of the patient based on the position information and biological information received by the antenna, **characterised in that** the display device (50) is adapted to be able to select to display or not to display the position of the patient, wherein
the display device (50) is configured to determine whether the state of the patient is normal or abnormal, based on the biological information transmitted from the medical transmitter (30),
when determining that the state of the patient is normal, the display device (50) is configured to not display the position of the patient, and
when determining that the state of the patient is abnormal, the display device (50) is configured to display the position of the patient.

2. The patient position display system (10) according to claim 1, **characterised in that**:
the position information transmitter (20a, 20b) is adapted to alternately repeat a transmission time period when the position information is transmitted, and a pause time period when the position information is not transmitted, and
the position information transmitter (20a, 20b) is adapted to switch between a gate mode in which the pause time period is shorter than a predetermined reference time period, and an area mode in which the pause time period is longer than the reference time period.

3. The patient position display system (10) according to claim 1 or 2, **characterised in that**:
the display device (50) is adapted to display the information indicating that the medical transmitter (30) moves outside a reception area of the antenna in case that the biological information is not received by the antenna within a predetermined time period before next position information is received, and wherein
the display device (50) is adapted to display the information indicating that a power supply of the medical transmitter is lost in case that the biological information is not received by the antenna after an elapse of the predetermined time period.

## Patentansprüche

1. Patientenposition-Anzeigesystem (10), umfassend:
einen Positionsinformationssender (20a, 20b), der angepasst ist, um Positionsinformationen über eine Position eines Ortes zu übertragen;
einen medizinischen Sender (30), der an einem Patienten anbringbar ist und angepasst ist, um biologische Informationen über den Patienten zu erfassen und zu senden, wobei der medizinische Sender angepasst ist, um die Positionsinformationen von dem Positionsinformationssender zu empfangen und die Positionsinformationen zu senden, wenn er die Positionsinformationen empfängt;
eine Antenne (40), die angepasst ist, um die Positionsinformationen und die biologischen Informationen von dem medizinischen Sender zu empfangen; und
eine Anzeigevorrichtung (50), die angepasst ist, um eine Position und einen Zustand des Patienten basierend auf den Positionsinformationen und den biologischen Informationen, die durch die Antenne empfangen werden, anzuzeigen,
**dadurch gekennzeichnet, dass** die Anzeigevorrichtung (50) angepasst ist, um in der Lage zu sein auszuwählen, die Position des Patienten anzuzeigen oder nicht anzuzeigen,
wobei die Anzeigevorrichtung (50) konfiguriert ist, um zu bestimmen, ob der Zustand des Patienten normal oder anormal ist, basierend auf den biologischen Informationen, die von dem medizinischen Sender (30) gesendet wurden,
wenn bestimmt wird, dass der Zustand des Patienten normal ist, die Anzeigevorrichtung (50) konfiguriert ist, um die Position des Patienten nicht anzuzeigen, und
wenn bestimmt wird, dass der Zustand des Patienten anormal ist, die Anzeigevorrichtung (50) konfiguriert ist, um die Position des Patienten anzuzeigen.

2. Patientenposition-Anzeigesystem (10) nach Anspruch 1, **dadurch gekennzeichnet, dass:**
der Positionsinformationssender (20a, 20b) angepasst ist, um abwechselnd einen Sendezeitraum, wenn die Positionsinformationen gesendet werden, und einen Pausenzeitraum, wenn die Positionsinformationen nicht gesendet werden, zu wiederholen, und
der Positionsinformationssender (20a, 20b) angepasst ist, um zwischen einem Gate-Modus, in welchem der Pausenzeitraum kürzer als ein vorgegebener Referenz-Zeitraum ist, und einem Bereich-Modus, in welchem der Pausenzeitraum länger als der Referenz-Zeitraum ist, zu wechseln.

3. Patientenposition-Anzeigesystem (10) nach Anspruch 1 oder 2, **dadurch gekennzeichnet, dass:**
die Anzeigevorrichtung (50) angepasst ist, um die Informationen anzuzeigen, die angeben, dass sich der medizinische Sender (30) außerhalb eines Empfangsbereiches der Antenne bewegt, falls die biologischen Informationen nicht durch die Antenne innerhalb eines vorgegebenen Zeitraums empfangen werden, bevor die nächsten Positionsinformationen empfangen werden, und wobei
die Anzeigevorrichtung (50) angepasst ist, um die Informationen anzuzeigen, die angeben, dass eine Stromversorgung des medizinischen Senders verloren ist, falls die biologischen Informationen nicht durch die Antenne nach einem Ablauf des vorgegebenen Zeitraums empfangen werden.

## Revendications

1. Système d'affichage de position de patient (10) comprenant :
un émetteur d'informations de position (20a, 20b) adapté pour émettre des informations de position sur une position d'une place ;
un émetteur médical (30) pouvant être attaché à un patient et adapté pour détecter et émettre des informations biologiques sur le patient, l'émetteur médical étant adapté pour recevoir les informations de position depuis l'émetteur d'informations de position et pour émettre les informations de position lors de la réception des informations de position ;
une antenne (40) adaptée pour recevoir les informations de position et les informations biologiques depuis l'émetteur médical ; et
un dispositif d'affichage (50) adapté pour afficher une position et un état du patient sur la base des informations de position et des informations biologiques reçues par l'antenne,
**caractérisé en ce que** le dispositif d'affichage (50) est adapté pour être capable de sélectionner ou d'afficher ou de ne pas afficher la position du patient, dans lequel le dispositif d'affichage (50) est configuré pour déterminer si l'état du patient est normal ou anormal, sur la base des informations biologiques émises depuis l'émetteur médical (30),
lors de la détermination du fait que l'état du patient est normal, le dispositif d'affichage (50) est configuré pour ne pas afficher la position du patient, et
lors de la détermination du fait que l'état du patient est anormal, le dispositif d'affichage (50) est configuré pour afficher la position du patient.

2. Système d'affichage de position de patient (10) selon la revendication 1, **caractérisé en ce que** :
l'émetteur d'informations de position (20a, 20b) est adapté pour répéter en alternance une période d'émission lorsque les informations de position sont émises, et une période de pause lorsque les informations de position ne sont pas émises, et
l'émetteur d'informations de position (20a, 20b) est adapté pour effectuer une commutation entre un mode porte dans lequel la période de pause est plus courte qu'une période de référence prédéterminée, et un mode zone dans lequel la période de pause est plus longue que la période de référence.

3. Système d'affichage de position de patient (10) selon la revendication 1 ou 2, **caractérisé en ce que** :
le dispositif d'affichage (50) est adapté pour afficher les informations indiquant que l'émetteur médical (30) se déplace à l'extérieur d'une zone de réception de l'antenne dans le cas où les informations biologiques ne sont pas reçues par l'antenne dans une période prédéterminée avant la réception d'informations de position suivantes, et dans lequel
le dispositif d'affichage (50) est adapté pour afficher les informations indiquant qu'une alimentation électrique de l'émetteur médical est perdue dans le cas où les informations biologiques ne sont pas reçues par l'antenne après l'écoulement d'une période prédéterminée.
